(19) Europäisches Patentamt / European Patent Office / Office européen des brevets

(11) **EP 4 174 088 A1**

(12) **EUROPEAN PATENT APPLICATION**
published in accordance with Art. 153(4) EPC

(43) Date of publication:
**03.05.2023 Bulletin 2023/18**

(21) Application number: **21834395.2**

(22) Date of filing: **29.06.2021**

(51) International Patent Classification (IPC):
**C07K 16/28** (2006.01)  **C07K 14/55** (2006.01)
**A61K 38/00** (2006.01)  **A61P 35/00** (2006.01)
**A61K 39/00** (2006.01)

(52) Cooperative Patent Classification (CPC):
**A61K 38/00; A61K 39/00; A61P 35/00;
C07K 14/55; C07K 16/28**

(86) International application number:
**PCT/KR2021/008198**

(87) International publication number:
**WO 2022/005174 (06.01.2022 Gazette 2022/01)**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(30) Priority: **30.06.2020  KR 20200079978**

(71) Applicant: **GI Innovation, Inc.
Seoul 05855 (KR)**

(72) Inventors:
• **JANG, Myung Ho
Seoul 05849 (KR)**
• **CHO, Young-Gyu
Daejeon 34032 (KR)**
• **OH, Young Min
Yongin-si Gyeonggi-do 16847 (KR)**
• **SHIM, Yaein
Seoul 05736 (KR)**

(74) Representative: **HGF
HGF Limited
1 City Walk
Leeds LS11 9DX (GB)**

Remarks:
The complete document including Reference Table(s) and the Sequence Listing(s) can be downloaded from the EPO website

(54) **FUSION PROTEIN COMPRISING ANTI-LAG-3 ANTIBODY AND IL-2, AND USE THEREOF**

(57)    The present invention relates to a fusion protein comprising an anti-LAG-3 antibody and IL-2 or a variant thereof. In the fusion protein according to the present invention, the anti-LAG-3 antibody moiety may bind to LAG-3, thereby regulating the binding of LAG-3 and MH-CII. Moreover, the IL-2 protein or a variant thereof may regulate the activity of T cells. Therefore, a pharmaceutical composition comprising the fusion protein increases the immune activity *in vivo* and can be effectively used as an anticancer agent, and thus is highly industrially applicable.

[FIG. 10]

EP 4 174 088 A1

## Description

## Technical Field

The present invention relates to a fusion protein comprising an anti-LAG-3 antibody and IL-2, and a use thereof. Specifically, the present invention relates to a novel fusion protein having the efficacy of treating or preventing cancer.

## Background Art

Cancer immunotherapy is a method for treating cancer using immune responses in the body. The cancer immunotherapy can induce an immune system to attack cancer cells by targeting antigens such as surface proteins of the cancer cells. In particular, it has been reported that anticancer immunity can be activated by blocking an immune checkpoint pathway. The immune checkpoint is one of the main mechanisms by which tumor cells cause immune evasion. Therefore, inhibiting or blocking the immune checkpoint can increase activation of T cells, and thus anti-tumor immune can be enhanced.

Meanwhile, IL-2 is synthesized mainly by activated T cells, in particular, CD4+ helper T cells. IL-2 stimulates proliferation and differentiation of T cells, and induces production of cytotoxic T lymphocytes (CTLs) and the differentiation of peripheral blood lymphocytes into cytotoxic cells and lymphokine activated killer cells (LAK cells).

However, IL-2 has a dual function in immune response in that it is important not only for mediating an increase in number of immune cells and activity thereof, but also for maintaining immune tolerance. In addition, it has been reported that IL-2 may not be optimal for inhibiting tumor growth. The reason is that in the presence of IL-2, activation-induced cell death (AICD) may occur in the resulting cytotoxic T lymphocytes, and the immune response may be inhibited by IL-2-dependent regulatory T cells (Treg) (Imai et al., Cancer Sci 98, 416 to 423, 2007).

Meanwhile, LAG-3 is known to have a mechanism similar to that of PD-1. LAG-3 is an immune checkpoint inhibitor expressed in T cells and NK cells, and has a structure similar to that of CD4. However, LAG-3 has 30 additional amino acids in a D1 domain and is thus known to have a high affinity with MHC class II (MHCII).

## Detailed Description of Invention

## Technical Problem

Accordingly, the present inventors have studied to develop a fusion protein having a new combination which enhances the activity of immune cells. As a result, the present inventors have confirmed that a fusion protein comprising an anti-LAG-3 antibody and an IL-2 variant effectively regulates immune cells. Based on the result, the present inventors have confirmed that the fusion protein is effective as an anticancer agent, thereby completing the present invention.

## Solution to Problem

In order to achieve the above object, in an aspect of the present invention, there is provided a fusion protein comprising an antibody that specifically binds to LAG-3 and an IL-2 protein or a variant thereof.

In another aspect of the present invention, there is provided a polynucleotide encoding the fusion protein, an expression vector including the polynucleotide, and a transformed cell into which the expression vector has been introduced.

In yet another aspect of the present invention, there is provided a method for preparing a fusion protein, the method including culturing the transformed cell; and collecting a fusion protein.

In still another aspect of the present invention, there is provided the fusion protein and a medical use thereof.

In still yet another aspect of the present invention, there is provided a use of the fusion protein for treating or preventing cancer.

In still yet another aspect of the present invention, there is provided a method for treating or preventing cancer, the method including administering the fusion protein to a subject.

In still yet another aspect of the present invention, there is provided a use of the fusion protein for the manufacture of a medicament for treatment or prevention of cancer.

## Effects of Invention

The fusion protein comprising an anti-LAG-3 antibody and an IL-2 variant, according to the present invention not only can regulate the mechanism related to LAG-3, but also have the same or similar function as IL-2. That is, the fusion protein not only can regulate the binding of LAG-3 and MHCII, but also activate immune cells. Therefore, the fusion protein can be used as an anticancer agent.

**Brief Description of Drawings**

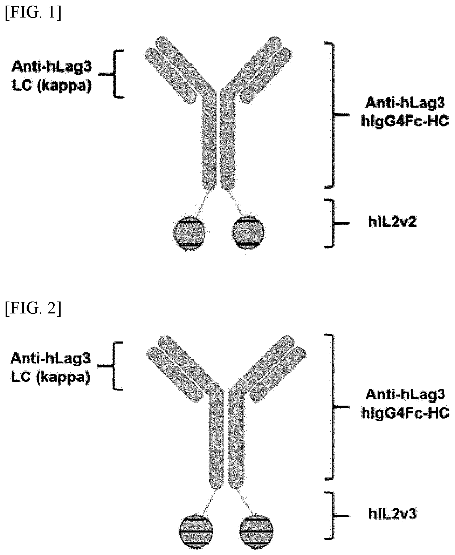

FIG. 1 illustrates the structure of an anti-hLAG-3 antibody-hIgG4 Fc-hIL-2v2 fusion protein (GI-104E1) as one embodiment.
FIG. 2 illustrates the structure of an anti-hLAG-3 antibody-hIgG4 Fc-hIL-2v3 fusion protein (GI-104E2) as one embodiment.
FIG. 3 is a picture in which GI-104E1 was produced and then confirm ed by SDS-PAGE.
FIG. 4 is a picture in which GI-104E2 was produced and then confirm ed by SDS-PAGE.
FIG. 5 is a picture in which GI-104E1 was produced and then confirm ed by western blot.
FIG. 6 is a picture in which GI-104E2 was produced and then confirm ed by western blot.
FIG. 7 illustrates the mechanism by which the action of fusion protein and experimental method of GI-104E1 or GI-104E2.
FIG. 8 is a graph for verifying, with LAG-3 blockade assay, whether GI-104E1 binds to LAG-3 and inhibits LAG-3-NMCII-mediated signaling.
FIG. 9 is a graph for verifying, with LAG-3 blockade assay, whether GI-104E2 binds to LAG-3 and inhibits LAG-3-MHCII-mediated signaling.
FIG. 10 is a graph showing tumor volumes observed by administering, to mice subcutaneously transplanted with CT26 cancer cells, a vehicle (PBS), an anti-LAG-3 antibody, Fc-IL-2v2, a combination of an anti-LAG-3 antibody and Fc-IL-2v2, and the fusion protein (GI-104E1) comprising anti-LAG-3 antibody and IL-2v2, respectively, once a week for three weeks.
FIG. 11 is a graph showing tumor volumes of the subjects after administering, to the mice subcutaneously transplanted with CT26 cancer cells, the vehicle (PBS), the anti-LAG-3 antibody, the Fc-IL-2v2, the combination of an anti-LAG-3 antibody and Fc-IL-2v2, and the fusion protein (GI-104E1) comprising anti-LAG-3 and IL-2v2, respectively.
FIG. 12 is a graph showing tumor volumes of the subjects of the group in which the vehicle (PBS) was administered to the mice subcutaneously transplanted with CT26 cancer cells.
FIG. 13 is a graph showing tumor volumes of the subjects of the group in which the anti-LAG-3 antibody was administered to the mice subcutaneously transplanted with CT26 cancer cells.
FIG. 14 is a graph showing tumor volumes of the subjects of the group in which the Fc-IL-2v2 was administered to the mice subcutaneously transplanted with CT26 cancer cells.
FIG. 15 is a graph showing tumor volumes of the subjects of the group in which the anti-LAG-3 antibody and the Fc-IL-2v2 were co-administered to the mice subcutaneously transplanted with CT26 cancer cells.
FIG. 16 is a graph showing tumor volumes of the subjects of the group in which the fusion protein (GI-104E1) comprising anti-LAG-3 and IL-2v2 was administered to the mice subcutaneously transplanted with CT26 cancer cells.

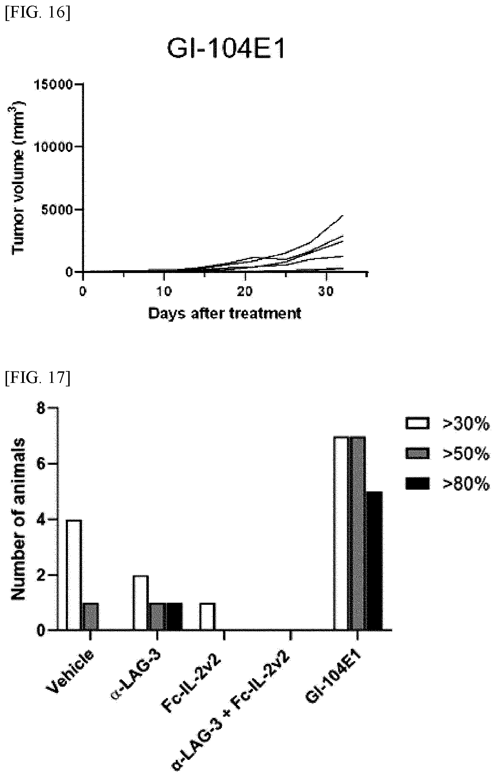

FIG. 17 is a graph showing the number of subjects, having tumor growth inhibition of 30% or greater, 50% or greater, and 80% or greater, of each group on the basis of the mean tumor volume growth of the group in which the vehicle (PBS) was administered, after administering the vehicle (PBS), the anti-LAG-3 antibody, the Fc-IL-2v2, the combination of an anti-LAG-3 antibody and Fc-IL-2v2, and the fusion protein (GI-104E1) comprising anti-LAG-3 and IL-2v2 to the mice subcutaneously transplanted with CT26 cancer cells, respectively, once a week for three weeks, and 32 days after the first administration.
FIG. 18 is a graph showing survival rates observed by administering, to the mice subcutaneously transplanted with CT26 cancer cells, the vehicle (PBS), the anti-LAG-3 antibody, the Fc-IL-2v2, the combination of an anti-LAG-3 antibody and Fc-IL-2v2, and the fusion protein (GI-104E1) comprising anti-LAG-3 and IL-2v2, respectively, once a week for three weeks.
FIG. 19 is a graph showing tumor volumes observed by administering, to mice subcutaneously transplanted with CT26 cancer cells, a vehicle (PBS), an anti-LAG-3 antibody, Fc-IL-2v3, a combination of an anti-LAG-3 antibody and Fc-IL-2v3, and a fusion protein (GI-104E2) comprising anti-LAG-3 and IL-2v3, respectively, once a week for three weeks.
FIG. 20 is a graph showing tumor volumes of subjects for each administration group after administering, to the mice subcutaneously transplanted with CT26 cancer cells, the vehicle (PBS), the anti-LAG-3 antibody, the Fc-IL-2v3, the combination of an anti-LAG-3 antibody and Fc-IL-2v3, and the fusion protein (GI-104E2) comprising anti-LAG-3 and IL-2v3, respectively.
FIG. 21 is a graph showing tumor volumes of the subjects of the group in which the vehicle (PBS) was administered to the mice subcutaneously transplanted with CT26 cancer cells.
FIG. 22 is a graph showing tumor volumes of the subjects of the group in which the anti-LAG-3 antibody was administered to the mice subcutaneously transplanted with CT26 cancer cells.

FIG. 23 is a graph showing tumor volumes of the subjects of the group in which the Fc-IL-2v3 was administered to the mice subcutaneously transplanted with CT26 cancer cells.

FIG. 24 is a graph showing tumor volumes of the subjects of the group in which the anti-LAG-3 antibody and the Fc-IL-2v3 were co-administered to the mice subcutaneously transplanted with CT26 cancer cells.

FIG. 25 is a graph showing tumor volumes of the subjects of the group in which the fusion protein (GI-104E2) comprising anti-LAG-3 and IL-2v3 was administered to the mice subcutaneously transplanted with CT26 cancer cells.

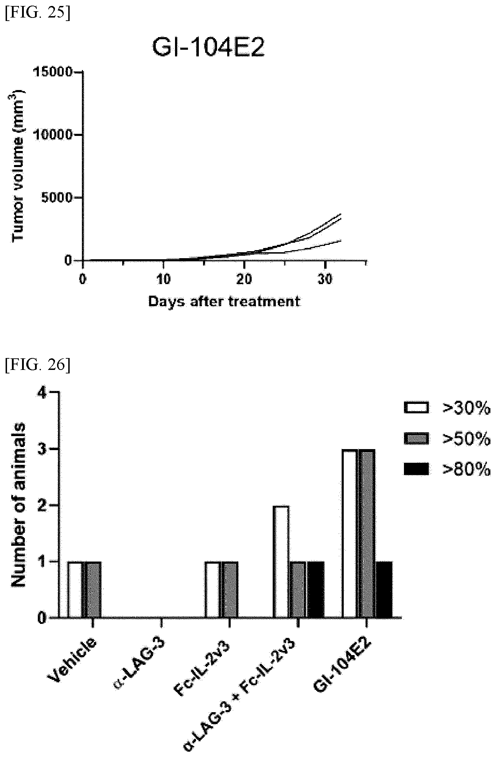

FIG. 26 is a graph showing the number of subjects, having tumor growth inhibition of 30% or greater, 50% or greater, and 80% or greater, of each group on the basis of the mean tumor volume growth of the group in which the vehicle (PBS) was administered, after administering the vehicle (PBS), the anti-LAG-3 antibody, the Fc-IL-2v3, the combination of an anti-LAG-3 antibody and Fc-IL-2v3, and the fusion protein (GI-104E2) comprising anti-LAG-3 and IL-2v3, respectively, to the mice subcutaneously transplanted with CT26 cancer cells once a week for three weeks, and 32 days after the first administration.

FIG. 27 is a graph showing survival rates observed by administering the vehicle (PBS), the anti-LAG-3 antibody, the Fc-IL-2v3, the combination of an anti-LAG-3 antibody and Fc-IL-2v3, and the fusion protein (GI-104E2) comprising anti-LAG-3 and IL-2v3, respectively, to the mice subcutaneously transplanted with CT26 cancer cells once a week for three weeks.

**Best Mode for Carrying out the Invention**

**Fusion protein comprising anti-LAG-3 antibody and IL-2**

In an aspect of the present invention, there is provided a fusion protein comprising an antibody that specifically binds to LAG-3 and an IL-2 protein.

Here, the fusion protein may be in a form in which at least one IL-2 protein is linked to the anti-LAG-3 antibody. In one embodiment, the fusion protein may contain one or two IL-2 proteins or variants thereof. Here, the anti-LAG-3 antibody and the IL-2 may be attached to each other via a linker.

As used herein, the term "LAG-3, referred to as CD223 or lymphocyte activation gene 3. The protein is encoded by a LAG-3 gene. LAG-3 is known to have a mechanism similar to that of PD-1. Moreover, LAG-3 is an immune checkpoint inhibitor expressed in T cells and NK cells, and has a structure similar to that of CD4. However, LAG-3 has 30 additional amino acids in a D1 domain and thus has a high affinity with MHC class II. Due to such structural features, LAG-3 is also known to inhibit T cell activation.

The anti-LAG-3 antibody in the present invention may be an antibody that specifically binds to the LAG-3. Moreover, a fragment of the antibody may be used in any form as long as the fragment contains an antigen-binding domain capable of specifically binding to LAG-3. Here, HCDR1, HCDR2, and HCDR3 of a heavy chain variable region of the anti-LAG-3 antibody may contain the amino acid sequences of SEQ ID NO: 1, SEQ ID NO: 2, and SEQ ID NO: 3, respectively. Moreover, LCDR1, LCDR2, and LCDR3 of a light chain variable region of the anti-LAG-3 antibody may contain the amino acid sequences of SEQ ID NO: 5, SEQ ID NO: 6, and SEQ ID NO: 7, respectively.

In an embodiment, the antibody that specifically binds to LAG-3 may include a heavy chain variable region containing the amino acid sequence of SEQ ID NO: 4, and a light chain variable region containing the amino acid sequence of SEQ ID NO: 8.

As used herein, the term "IL-2" or "interleukin-2", unless otherwise stated, refers to any wild-type IL-2 obtained from any vertebrate source, including mammals, for example, primates (such as humans) and rodents (such as mice and rats). IL-2 may be obtained from animal cells, and also includes one obtained from recombinant cells capable of producing IL-2. In addition, IL-2 may be wild-type IL-2 or a variant thereof.

In the present specification, IL-2 or a variant thereof may be collectively expressed by the term "IL-2 protein" or "IL-2 polypeptide." IL-2, an IL-2 protein, an IL-2 polypeptide, and an IL-2 variant specifically bind to, for example, an IL-2 receptor. This specific binding may be identified by methods known to those skilled in the art.

The IL-2 may be in a mature form. Specifically, the mature IL-2 may not contain a signal sequence, and may contain a fragment of wild-type IL-2 in which a portion of the N-terminus or C-terminus of wild-type IL-2 is truncated. Here, the IL-2 may have the amino acid sequence of SEQ ID NO: 22.

As used herein, the term "IL-2 variant" refers to a form in which a portion of amino acids in the full-length IL-2 or the above-described fragment of IL-2 is substituted. That is, an IL-2 variant may have an amino acid sequence different from wild-type IL-2 or a fragment thereof. However, an IL-2 variant may have activity equivalent or similar to the wild-type IL-2. Here, "IL-2 activity" may, for example, refer to specific binding to an IL-2 receptor, which specific binding may be measured by methods known to those skilled in the art.

Specifically, an IL-2 variant may be obtained by substitution of a portion of amino acids in the wild-type IL-2. An embodiment of the IL-2 variant obtained by amino acid substitution may be obtained by substitution of at least one

of the 38th, 42nd, 45th, 61st, and 72nd amino acids in the amino acid sequence of SEQ ID NO: 22.

Specifically, the IL-2 variant may be obtained by substitution of at least one of the 38th, 42nd, 45th, 61st, or 72nd amino acid in the amino acid sequence of SEQ ID NO: 22 with another amino acid. According to an embodiment, one, two, or three amino acids may be substituted as long as such IL-2 variant maintains IL-2 activity.

In an embodiment, an IL-2 variant may be in a form in which two amino acids are substituted. Specifically, the IL-2 variant may be obtained by substitution of the 38th and 42nd amino acids in the amino acid sequence of SEQ ID NO: 22. In addition, in an embodiment, the IL-2 variant may be obtained by substitution of the 38th and 45th amino acids in the amino acid sequence of SEQ ID NO: 22. In addition, In an embodiment, the IL-2 variant may be obtained by substitution of the 38th and 61st amino acids in the amino acid sequence of SEQ ID NO: 22. In addition, in an embodiment, the IL-2 variant may be obtained by substitution of the 38th and 72nd amino acids in the amino acid sequence of SEQ ID NO: 22. In addition, in an embodiment, the IL-2 variant may be obtained by substitution of the 42nd and 45th amino acids in the amino acid sequence of SEQ ID NO: 22. In addition, in an embodiment, the IL-2 variant may be obtained by substitution of the 42nd and 61st amino acids in the amino acid sequence of SEQ ID NO: 22. In addition, in an embodiment, the IL-2 variant may be obtained by substitution of the 42nd and 72nd amino acids in the amino acid sequence of SEQ ID NO: 22. In addition, in an embodiment, the IL-2 variant may be obtained by substitution of the 45th and 61st amino acids in the amino acid sequence of SEQ ID NO: 22. In addition, in an embodiment, the IL-2 variant may be obtained by substitution of the 45th and 72nd amino acids in the amino acid sequence of SEQ ID NO: 22. In addition, in an embodiment, the IL-2 variant may be obtained by substitution of the 61st and 72nd amino acids in the amino acid sequence of SEQ ID NO: 22.

Furthermore, an IL-2 variant may be in a form in which three amino acids are substituted. Specifically, the IL-2 variant may be obtained by substitution of the 38th, 42nd, and 45th amino acids in the amino acid sequence of SEQ ID NO: 22. In addition, in an embodiment, the IL-2 variant may be obtained by substitution of the 38th, 42nd, and 61st amino acids in the amino acid sequence of SEQ ID NO: 22. In addition, in an embodiment, the IL-2 variant may be obtained by substitution of the 38th, 42nd, and 72nd amino acids in the amino acid sequence of SEQ ID NO: 22. In addition, in an embodiment, the IL-2 variant may be obtained by substitution of the 38th, 45th, and 61st amino acids in the amino acid sequence of SEQ ID NO: 22. In addition, in an embodiment, the IL-2 variant may be obtained by substitution of the 38th, 45th, and 72nd amino acids in the amino acid sequence of SEQ ID NO: 22. In one embodiment, the IL-2 variant may be obtained by substitution of the 38th, 61st, and 72nd amino acids in the amino acid sequence of SEQ ID NO: 22. In addition, in an embodiment, the IL-2 variant may be obtained by substitution of the 42nd, 45th, and 61st amino acids in the amino acid sequence of SEQ ID NO: 22. In addition, in an embodiment, the IL-2 variant may be obtained by substitution of the 42nd, 45th, and 72nd amino acids in the amino acid sequence of SEQ ID NO: 22. In addition, in an embodiment, the IL-2 variant may be obtained by substitution of the 45th, 61st, and 72nd amino acids in the amino acid sequence of SEQ ID NO: 22.

Here, the "another amino acid" introduced by the substitution may be any one selected from the group consisting of alanine, arginine, asparagine, aspartic acid, cysteine, glutamic acid, glutamine, histidine, isoleucine, leucine, lysine, methionine, phenyl alanine, proline, serine, threonine, tryptophan, tyrosine, and valine. However, regarding the amino acid substitution for the IL-2 variant, in the amino acid sequence of SEQ ID NO: 22, the 38th amino acid cannot be substituted with arginine, the 42nd amino acid cannot be substituted with phenyl alanine, the 45th amino acid cannot be substituted with tyrosine, the 61st amino acid cannot be substituted with glutamic acid, and the 72nd amino acid cannot be substituted with leucine.

Regarding amino acid substitution for an IL-2 variant, in the amino acid sequence of SEQ ID NO: 22, the 38th amino acid, arginine, may be substituted with an amino acid other than arginine. Preferably, regarding amino acid substitution for an IL-2 variant, in the amino acid sequence of SEQ ID NO: 22, the 38th amino acid, arginine, may be substituted with alanine (R38A).

Regarding amino acid substitution for an IL-2 variant, in the amino acid sequence of SEQ ID NO: 22, the 42nd amino acid, phenylalanine, may be substituted with an amino acid other than phenylalanine. Preferably, regarding amino acid substitution for an IL-2 variant, in the amino acid sequence of SEQ ID NO: 22, the 42nd amino acid, phenylalanine, may be substituted with alanine (F42A).

Regarding amino acid substitution for an IL-2 variant, in the amino acid sequence of SEQ ID NO: 22, the 45th amino acid, tyrosine, may be substituted with an amino acid other than tyrosine. Preferably, regarding amino acid substitution for an IL-2 variant, in the amino acid sequence of SEQ ID NO: 22, the 45th amino acid, tyrosine, may be substituted with alanine (Y45A).

Regarding amino acid substitution for an IL-2 variant, in the amino acid sequence of SEQ ID NO: 22, the 61st amino acid, glutamic acid, may be substituted with an amino acid other than glutamic acid. Preferably, regarding amino acid substitution for an IL-2 variant, in the amino acid sequence of SEQ ID NO: 22, the 61st amino acid, glutamic acid, may be substituted with arginine (E61R).

Regarding amino acid substitution for an IL-2 variant, in the amino acid sequence of SEQ ID NO: 22, the 72nd amino acid, leucine, may be substituted with an amino acid other than leucine. Preferably, regarding amino acid substitution for an IL-2 variant, in the amino acid sequence of SEQ ID NO: 22, the 72nd amino acid, leucine, may be substituted with glycine (L72G).

Specifically, an IL-2 variant may be obtained by at least one substitution selected from the group consisting of R38A, F42A, Y45A, E61R, and L72G, in the amino acid sequence of SEQ ID NO: 22. Preferably, an IL-2 variant may have amino acid substitutions at two or three positions among positions selected from the group consisting of R38A, F42A, Y45A, E61R, and L72G.

An IL-2 variant may be in a form in which two amino acids are substituted. Specifically, an IL-2 variant may be obtained by the substitutions, R38A and F42A. In addition, in an embodiment, an IL-2 variant may be obtained by the substitutions, R38A and Y45A. In addition, in an embodiment, an IL-2 variant may be obtained by the substitutions, R38A and E61R. In addition, in an embodiment, an IL-2 variant may be obtained by the substitutions, R38A and L72G. In addition, in an embodiment, an IL-2 variant may be obtained by the substitutions, F42A and Y45A. In addition, in an embodiment, an IL-2 variant may be obtained by the substitutions, F42A and E61R. In addition, in an embodiment, an IL-2 variant may be obtained by the substitutions, F42A and L72G. In addition, in an embodiment, an IL-2 variant may be obtained by the substitutions, E61R and L72G.

Furthermore, an IL-2 variant may be in a form in which three amino acids are substituted. Specifically, an IL-2 variant may be obtained by the substitutions, R38A, F42A, and Y45A. In addition, in an embodiment, an IL-2 variant may be obtained by the substitutions, R38A, F42A, and E61R. In addition, in an embodiment, an IL-2 variant may be obtained by the substitutions, R38A, F42A, and L72G. In addition, in an embodiment, an IL-2 variant may be obtained by the substitutions, R38A, Y45A, and E61R. In addition, in an embodiment, an IL-2 variant may be obtained by the substitutions, R38A, Y45A, and L72G. In addition, in an embodiment, an IL-2 variant may be obtained by the substitutions, F42A, Y45A, and E61R. In addition, in an embodiment, an IL-2 variant may be obtained by the substitutions, F42A, Y45A, and L72G. In addition, in an embodiment, an IL-2 variant may be obtained by the substitutions, F42A, E61R, and L72G. In addition, in an embodiment, an IL-2 variant may be obtained by the substitutions, Y45A, E61R, and L72G.

In an embodiment, an IL-2 variant may have the amino acid sequence of SEQ ID NO: 20 or SEQ ID NO: 21.

In addition, an IL-2 variant may be characterized by having low *in vivo* toxicity. Here, the low *in vivo* toxicity may be a side effect caused by binding of IL-2 to the IL-2 receptor α-chain (IL-2Rα). Various IL-2 variants have been developed to ameliorate the side effect caused by the binding of the IL-2 to IL-2Rα, and such IL-2 variants may be those disclosed in US Patent No. 5,229,109 and Korean Patent No. 10-1667096. In particular, IL-2 variants described in the present application have low binding ability for the IL-2 receptor alpha chain (IL-2Rα) and thus have lower *in vivo* toxicity than the wild-type IL-2.

The fusion protein may include an immunoglobulin Fc region. Here, an Fc domain of the immunoglobulin includes a heavy chain constant region 2 (CH2) and a heavy chain constant region 3 (CH3) of the immunoglobulin. The immunoglobulin may be IgG, IgA, IgE, IgD, or IgM, and preferably IgG4.

In addition, the Fc domain of an immunoglobulin may be an Fc domain variant as well as a wild-type Fc domain. In addition, as used herein, the term "Fc domain variant", may refers to a form which is different from the wild-type F domain in terms of glycosylation pattern, has a high glycosylation as compared with the wild-type Fc domain, or has a low glycosylation as compared with the wild-type Fc domain, or a deglycosylated form. In addition, an aglycosylated Fc domain is included therein. The Fc domain or a variant thereof may be adapted to have an adjusted number of sialic acids, fucosylations, or glycosylations, through culture conditions or genetic manipulation of a host.

In addition, glycosylation of the Fc domain of an immunoglobulin may be modified by conventional methods, such as a chemical method, enzymatic method, and genetic engineering method using microorganisms. In addition, the Fc domain variant may be in a mixed form of respective Fc regions of immunoglobulin, IgG, IgA, IgE, IgD, or IgM. In addition, the Fc domain variant may be in a form in which some amino acids of the Fc domain are substituted with other amino acids.

In an embodiment, a Fc domain may have the amino acid sequence of SEQ ID NO: 14.

The fusion protein according to the present invention may contain a fusion protein including a light chain variable region and a light chain constant region (CL1) of the anti-LAG-3 antibody, and a heavy chain variable region, a heavy chain constant region (CH1) of the anti-LAG-3 antibody, an Fc domain, and an IL-2 protein. Here, at least one IL-2 protein may be contained in the anti-LAG-3 antibody. In an embodiment, the fusion protein contains two IL-2 proteins. In addition, in an embodiment, IL-2 protein may be in a form of being linked to the C-terminus of the anti-LAG-3 antibody.

Specifically, the fusion protein containing the Fc domain and the IL-2 protein may be a dimer in which two fusion proteins represented by Structural Formula (1) are linked.

N'-X-[linker (1)]o-Fc region fragment or variant thereof-[linker (2)]p-Y-C' (1)

Here, in Structural Formula (1),

N' is the N-terminus of the fusion protein,

C' is the C-terminus of the fusion protein,

X is an anti-LAG-3 antibody or a fragment thereof,

Y is an IL-2 protein,

the linker (1) and the linker (2) are peptide linkers, and

o and p are each independently 0 or 1.

Here, the anti-LAG-3 antibody and the IL-2 protein are as described above. In addition, in the fusion protein, the IL-2 or a variant thereof may be linked to an Fc region linked to the C-terminus of the anti-LAG-3 antibody. Here, the IL-2 or a variant thereof and the Fc region may be linked by a linker.

The peptide linker (1) may consist of 1 to 50 contiguous amino acids, 3 to 30 contiguous amino acids, or 5 to 15 amino acids. In an embodiment, the peptide linker (1) may consist of 12 amino acids. In addition, the peptide linker (1) may contain at least one cysteine. Specifically, the peptide linker (1) may contain one, two, or three cysteines. In addition, the peptide linker (1) may be derived from the hinge of an immunoglobulin. In an embodiment, the peptide linker (1) may be a peptide linker consisting of the amino acid sequence of SEQ ID NO: 13.

The peptide linker (2) may consist of 1 to 30 contiguous amino acids, 2 to 20 contiguous amino acids, or 2 to 10 amino acids. In an embodiment, the peptide linker (2) may be (G4S)n (here, n is an integer of 1 to 10). Here, in (G4S)n, n may be 1, 2, 3, 4, 5, 6, 7, 8, 9, or 10. In an embodiment, the peptide linker may be a peptide linker consisting of the amino acid sequence of SEQ ID NO: 19.

Furthermore, the fusion protein represented by Structural Formula (1) may include proteins represented by Structural Formula (1-1) and Structural Formula (1-2).

$$\text{N'-X'-[linker (1)]o-Fc region fragment or variant thereof-[linker (2)]p-Y-C'} \qquad (1-1)$$

$$\text{N'-X''-C'} \qquad (1-2)$$

Here, in the Structural Formulaes (1-1) and (1-2),

N' is the N-terminus of the fusion protein,

C' is the C-terminus of the fusion protein,

X' is a heavy chain region of an anti-LAG-3 antibody, and includes a heavy chain variable region and CH1,

X" is a light chain region of an anti-LAG-3 antibody, and includes a light chain variable region and CL,

Y is an IL-2 protein,

the linker (1) and the linker (2) are peptide linkers, and

o and p are each independently 0 or 1.

Moreover, the heavy chain variable region and the light chain variable region are as described above.

The amino acid sequence of each of the regions constituting the fusion protein is as shown in Tables 1 and 2 below. Specifically, Table 1 shows the amino acid sequence for anti-hLAG-3(1E09)VL-kappa+CL. Table 2 shows the amino acid sequences of anti-hLAG-3(1E09)VH+CH1, hIgG4Fc, and hIL-2v2/hIL-2v3.

[Table 1]

| Classification | Amino acid sequence | SEQ ID NO. |
|---|---|---|
| Anti-hLAG-3(1E09)VL-kappa+CL | DIQMTQSPSSLSPSVGDRVTITCQASQEISIYLNWYQ QKPGKAPKLLIYDASNLETGVPSRFSGSGSGTDFTLTI SSLQPEDFATYYCQQTYITPYTFGQGTKLDIK RTVAAPSVFIFPPSDEQLKSGTASVVCLLNNFYPREA KVQWKVDNALQSGNSQESVTEQDSKDSTYSLSSTLT LSKADYEKHKVYACEVTHQGLSSPVTKSFNRGEC | 16 |

[Table 2]

| Classification | Amino acid sequence | SEQ ID NO. |
|---|---|---|
| Anti-hLAG-3(1E09) VH+CH1 | QVQLVQSGGDLVKPGGSLRLSCAASGFSFSDHYMN WIRQAPGKGLEWVAYIDTSATYIYYADSVKGRFTIS RDNAKNSLYLQMNSLRAEDTAVYYCARDNWGSLD YWGQGALVTVSS ASTKGPSVFPLAPCSRSTSESTAALGCLVKDYFPEPV TVSWNSGALTSGVHTFPAVLQSSGLYSLSSVVTVPSS SLGTKTYTCNVDHKPSNTKVDKRV | 12 |
| First linker | AESKYGPPCPPCP | 13 |
| F02(hIgG4Fc) | APEAAGGPSVFLFPPKPKDQLMISRTPEVTCVVVDVS QEDPEVQFNWYVDGVEVHNAKTKPREEQFNSTYRV VSVLTVLHQDWLNGKEYKCKVSNKGLPSSIEKTISK AKGQPREPQVYTLPPSQEEMTKNQVSLTCLVKGFYP SDIAVEWESNGQPENNYKTTPPVLDSDGSFFLYSRLT VDKSRWQEGNVFSCSVLHEALHNHYTQKSLSLSLG | 14 |
| Second linker | GGGGS | 19 |
| hIL-2v2 | APTSSSTKKTQLQLEHLLLDLQMILNGINNYKNPKLT AMLTAKFYMPKKATELKHLQCLEEELKPLEEVLNL AQSKNFHLRPRDLISNINVIVLELKGSETTFMCEYAD ETATIVEFLNRWITFCQSIISTLT | 20 |
| hIL-2v3 | APTSSSTKKTQLQLEHLLLDLQMILNGINNYKNPKLT AMLTAKFYMPKKATELKHLQCLERELKPLEEVLNL AQSKNFHLRPRDLISNINVIVLELKGSETTFMCEYAD ETATIVEFLNRWITFCQSIISTLT | 21 |
| IL-2 | APTSSSTKKTQLQLEHLLLDLQMILNGINNYKNPKLT RMLTFKFYMPKKATELKHLQCLEEELKPLEEVLNLA QSKNFHLRPRDLISNINVIVLELKGSETTFMCEYADE TATIVEFLNRWITFCQSIISTLT | 22 |

(continued)

| Classification | Amino acid sequence | SEQ ID NO. |
|---|---|---|
| IgG4 Fc-linker-IL-2v2 | AESKYGPPCPPCPAPEAAGGPSVFLFPPKPKDQLMIS RTPEVTCVVVDVSQEDPEVQFNWYVDGVEVHNAKT KPREEQFNSTYRVVSVLTVLHQDWLNGKEYKCKVS NKGLPSSIEKTISKAKGQPREPQVYTLPPSQEEMTKN QVSLTCLVKGFYPSDIAVEWESNGQPENNYKTTPPV LDSDGSFFLYSRLTVDKSRWQEGNVFSCSVLHEALH NHYTQKSLSLSLG GGGGS APTSSSTKKTQLQLEHLLLDLQMILNGINNYKNPKLT AMLTAKFYMPKKATELKHLQCLEEELKPLEEVLNL AQSKNFHLRPRDLISNINVIVLELKGSETTFMCEYAD ETATIVEFLNRWITFCQSIISTLT | 11 |
| IgG4 Fc-linker-IL-2v3 | AESKYGPPCPPCPAPEAAGGPSVFLFPPKPKDQLMIS RTPEVTCVVVDVSQEDPEVQFNWYVDGVEVHNAKT KPREEQFNSTYRVVSVLTVLHQDWLNGKEYKCKVS NKGLPSSIEKTISKAKGQPREPQVYTLPPSQEEMTKN QVSLTCLVKGFYPSDIAVEWESNGQPENNYKTTPPV LDSDGSFFLYSRLTVDKSRWQEGNVFSCSVLHEALH NHYTQKSLSLSLG GGGGS APTSSSTKKTQLQLEHLLLDLQMILNGINNYKNPKLT AMLTAKFYMPKKATELKHLQCLERELKPLEEVLNL AQSKNFHLRPRDLISNINVIVLELKGSETTFMCEYAD ETATIVEFLNRWITFCQSIISTLT | 24 |

**Polynucleotide encoding fusion protein**

In another aspect of the present invention, there is provided a polynucleotide encoding the fusion protein.

Here, the polynucleotide may contain nucleotide sequences encoding the fusion proteins represented by Structural Formula (1-1) and Structural Formula (1-2).

Specifically, a polynucleotide encoding the heavy chain region among the polynucleotides may contain the nucleotide sequence of SEQ ID NO: 17 or SEQ ID NO: 23. Moreover, a polynucleotide encoding the light chain region may contain SEQ ID NO: 18.

If the polynucleotides encode the same polypeptide, one or more nucleotides may be altered by substitution, deletion, insertion, or a combination thereof. When a nucleotide sequence is prepared by chemical synthesis, synthesis method well known in the art may be used, such as those described in Engels and Uhlmann (Angew Chem Int Ed Engl., 37:73-127, 1988). Such mathods may include triester, phosphite, phosphoramidite, and H-phosphate, PCR and other autoprimer methods, oligonucleotide syntheses on solid supports, and the like.

According to an embodiment, the polynucleotide may contain a nucleic acid sequence having an identify, to SEQ ID NO: 17, 18, or 23, of at least about 70%, at least about 75%, at least about 80%, at least about 85%, at least about 86%, at least about 87%, at least about 88%, at least about 89%, at least about 90%, at least about 91%, at least about 92%, at least about 93%, at least about 94%, at least about 95%, at least about 96%, at least about 97%, at least about 98%, at least about 99%, or at least about 100%.

The polynucleotide may further contain a nucleic acid encoding a signal sequence or a leader sequence. As used herein, the term "signal sequence" refers to a signal peptide that directs secretion of a target protein. The signal peptide is translated and then cleaved in a host cell. Specifically, the signal sequence is an amino acid sequence that initiates migration of a protein across the endoplasmic reticulum (ER) membrane.

Signal sequences are well known in the art for their characteristics. Such signal sequences typically contain 16 to 30 amino acid residues, and may contain more or fewer amino acid residues than such amino acid residues. A typical signal peptide is composed of three regions, that is, a basic N-terminal region, a central hydrophobic region, and a more polar C-terminal region. The central hydrophobic region contains 4 to 12 hydrophobic residues that cause the signal sequence to be immobilized during migration of an immature polypeptide through the membrane lipid bilayer.

After initiation, signal sequence is cleaved in the lumen of ER by cellular enzymes, commonly known as signal peptidases. Here, the signal sequence may be a secretory signal sequence of tPa (tissue Plasminogen Activation), HSV gDs (Herpes simplex virus glycoprotein D), an IgG signal sequence, or a growth hormone. Preferably, a secretory signal sequence used in higher eukaryotic cells including mammals and the like may be used. In addition, a signal sequence included in wild-type IL-2 may be used, or a signal sequence that has been substituted with a codon having high expression frequency in a host cell may be used. An embodiment thereof may include a light chain signal sequence of a 14.18 antibody (Gillies et al., J. Immunol. Meth 1989. 125: 191-202), a heavy chain signal sequence of a MOPC141 antibody (Sakano et al., Nature, 1980. 286: 676-683), and other signal sequences known in the art (see, for example, Watson et al., Nucleic Acid Research, 1984. 12:5145-5164).

**Vector carrying polynucleotide encoding fusion protein**

In yet another aspect of the present invention, there is provided a vector including the polynucleotide.

Here, the polynucleotide encoding the heavy chain region and the polynucleotide encoding the light chain region may be contained on different vectors. Alternatively, the polynucleotide encoding the heavy chain region and the polynucleotide encoding the light chain region may be contained on one vector.

The polynucleotide is as described above. Here, the polynucleotide encoding the heavy chain region may contain the nucleotide sequence of SEQ ID NO: 17 or 23, and the polynucleotide encoding the light chain region may contain the nucleotide sequence of SEQ ID NO: 18. In an embodiment, the polynucleotide encoding the heavy chain region and the polynucleotide encoding the light chain region may contain each the nucleotide sequence of SEQ ID NO: 17 and SEQ ID NO: 18. In an embodiment, the polynucleotide encoding the heavy chain region and the polynucleotide encoding the light chain region may contain each the nucleotide sequence of SEQ ID NO: 23 and SEQ ID NO: 18. Here, the vector may be two vectors containing each of polynucleotide combinations of the heavy chain and the light chain, or a bicistronic vector containing both polynucleotides of the combinations.

The vector may be introduced into a host cell to be recombined with and inserted into the genome of the host cell. Alternatively, the vector is understood as nucleic acid means containing a polynucleotide sequence which is autonomously replicated as an episome. Here, the vector may be operably linked to an appropriate promoter so that the polynucleotide may be expressed in a host cell. The vectors include linear nucleic acid, plasmid, phagemid, cosmid, RNA vector, viral vector, mini-chromosome, and analogs thereof. Examples of the viral vector include but are not limited to, retrovirus, adenovirus, and adeno-associated virus.

Specifically, the vector may include plasmid DNA, phage DNA, and the like, and commercially developed plasmids (pUC18, pBAD, pIDTSAMRT-AMP, or the like), *Escherichia* coli-derived plasmids (pYG601BR322, pBR325, pUC118, pUC119, or the like), *Bacillus subtilis*-derived plasmids (pUB110, pTP5, or the like), yeast-derived plasmids (YEp13, YEp24, YCp50, or the like), phage DNA (Charon4A, Charon21A, EMBL3, EMBL4, λgt10, λgt11, λZAP, or the like), animal viral vectors (a retrovirus, an adenovirus, a vaccinia virus, or the like), insect virus vectors (a baculovirus or the like). Since the vector exhibits different expression levels and modifications of a protein depending on the host cell, it is preferable to select and use a host cell most suitable for the purpose.

As used herein, the term "gene expression" or "expression" of the target protein is understood to mean transcription of DNA sequences, translation of mRNA transcripts, and secretion of a fusion protein products or fragments thereof. A useful expression vector may be RcCMV (Invitrogen, Carlsbad) or a variant thereof. The expression vector may further contain human cytomegalovirus (CMV) promoter for promoting continuous transcription of a target gene in mammalian cells, and a bovine growth hormone polyadenylation signal sequence for increasing the steady-state level of RNA after transcription.

**Transformed cell expressing fusion protein**

In still another aspect of the present invention, there is provided a transformed cell into which the vector is introduced.

Host cell for the transformed cell may include, but are not limited to, prokaryotic cells, eukaryotic cells, and cells of mammalian, plant, insect, fungal, or bacterial origin. As an example of the prokaryotic cells, *E. coli* may be used. In addition, as an example of the eukaryotic cells, yeast may be used. In addition, for the mammalian cells, CHO cells, F2N cells, COS cells, BHK cells, Bowes melanoma cells, HeLa cells, 911 cells, AT1080 cells, A549 cells, SP2/0 cells, human lymphoblastoids, NSO cells, HT-1080 cells, PERC.6 cells, HEK 293 cells, HEK293T cells, or the like may be used. However, the mammalian cells are not limited thereto, and any cells which are known to those skilled in the art to be usable as a mammalian host cells may all be used.

In addition, for the introduction of an expression vector into the host cell, CaCl$_2$ precipitation, Hanahan method whose efficiency has been increased efficiency by using a reducing agent such as dimethyl sulfoxide (DMSO) in the CaCl$_2$ precipitation, electroporation, calcium phosphate precipitation, protoplast fusion, agitation using silicon carbide fiber, Agrobacterium-mediated transformation, transformation using PEG, dextran sulfate, lipofectamine, and a dry/inhibition-mediated transformation, or the like may be used. In addition, a target may be delivered into a cell using infection as a means and using viral particles. In addition, the vector is introduced into the host cell, gene bombardment or the like may be used.

As described above, for optimization of properties of a fusion protein as a therapeutic agent, or for other purpose, glycosylation pattern of the fusion protein (for example, sialic acids, fucosylations, and glycosylations) may be adjusted by manipulating, through methods known to those skilled in the art, glycosylation-related genes possessed by host cells.

## Method for preparing fusion protein

In still yet another aspect of the present invention, there is provided a method for preparing a fusion protein comprising an anti-LAG-3 antibody and IL-2 or a variant thereof, the method comprising culturing the transformed cells; and collecting the aforementioned fusion protein from the culturemedium.

As used herein, the term "culturing", refers to a method for artificially growing microorganisms under appropriately controlled environmental conditions.

Culturing the transformed cells may be carried out using methods well known in the art. Specifically, the culturing is not particularly limited as long as the fusion protein of the present invention may be produced by expression. Specifically, the culture may be carried out in a batch process, or carried out continuously in a fed batch or repeated fed batch process.

In addition, the collecting the fusion protein from the cultured matter may be performed by a method known in the art. Specifically, the collection method is not particularly limited as long as the produced fusion protein according to the present invention may be collected. Preferably, the collection method may be a method such as centrifugation, filtration, extraction, spraying, drying, evaporation, precipitation, crystallization, electrophoresis, fractional dissolution (for example, ammonium sulfate precipitation), or chromatography (for example, ion-exchange, affinity, hydrophobicity, and size exclusion).

## Use of fusion protein

In still yet another aspect of the present invention, there is provided a pharmaceutical composition for treating or preventing cancer comprising the fusion protein.

As used herein, the term "cancer" is classified as a disease in which normal tissue cells proliferate unlimitedly for some reason and continue to develop rapidly regardless of the living phenomenon of the living body or the surrounding tissue state. The cancer in the present invention may be any one cancer selected from the group consisting of various cancers of the human body, such as gastric cancer, liver cancer, lung cancer, colorectal cancer, breast cancer, prostate cancer, ovarian cancer, pancreatic cancer, cervical cancer, thyroid cancer, laryngeal cancer, acute myeloid leukemia, brain tumor, neuroblastoma, retinoblastoma, head and neck cancer, salivary gland cancer, and lymphoma, but is not limited to the above types. In addition, for the purposes of the present invention, it may be a cancer that is resistant to radiation, but is not limited thereto.

As used herein, the term "prevention" refers to any action that inhibits the occurrence of cancer or delays the onset of cancer through the administration of the pharmaceutical composition. As used herein, the term "treatment" refers to any action that improves or beneficially changes the symptoms of cancer through the administration of the pharmaceutical composition.

The pharmaceutical composition may further comprise a pharmaceutically acceptable carrier. The pharmaceutically acceptable carrier may be any carrier as long as the carrier is a non-toxic substance suitable for delivery to a patient. Distilled water, alcohol, fat, wax, and inert solid may be contained as the carrier. A pharmaceutically acceptable adjuvant (buffer or dispersant) may also be contained in the pharmaceutical composition.

Specifically, by including a pharmaceutically acceptable carrier, the pharmaceutical composition may be prepared into a parenteral formulation epending on its route of administration using conventional method known in the art. Here, the term "pharmaceutically acceptable" means that the carrier does not have more toxicity than the subject to be applied (prescribed) can adapt while not inhibiting activity of the active ingredient.

When the pharmaceutical composition is prepared into a parenteral formulation, it may be made into preparations in the form of injections, a transdermal patch, nasal inhalants, or a suppositories with suitable carriers according to methods known in the art.In a case of being made into injections, sterile water, ethanol, polyol such as glycerol or propylene glycol, or a mixture thereof may be used as a suitable carrier, and an isotonic solution, such as Ringer's solution, phosphate buffered saline (PBS) containing triethanol amine or sterile water for injection, and 5% dextrose, or the like may preferably be used. Formulation of the pharmaceutical composition is known in the art, and reference may specifically be made to Remington's Pharmaceutical Sciences (19th ed., 1995) and the like. The document is considered part of the present specification.

On the other hand, the pharmaceutical composition of the present invention is administered in a pharmaceutically effective amount. As used herein, the term "administration" refers to introducing a predetermined substance to a subject by an appropriate method, and the route of administration of the composition may be through any general route as long as it may reach a target tissue. Their may be intraperitoneal administration, intravenous administration, intramuscular administration, subcutaneous administration, intradermal administration, topical administration, intranasal administration, or intrarectal administration, but is not limited thereto.

The term "subject" refers to all animals including a human, a rat, a mouse, a livestock, and the like. Preferably, it may be a mammal including a human.

The term "pharmaceutically effective amount" refers to an amount that is sufficient to treat a disease with a reasonable benefit/risk ratio applicable to a medical treatment, and does not cause side effects, and the effective dose level may be readily determined by one of ordinaty skill in the art according to factors including the sex, age, body weight, and health condition of the patient, the type and severity of the disease, the activity of the drug, the sensitivity to the drug, the administration method, the administration time, the route of administration, the excretion rate, the duration of treatment, drugs used in combination or concurrently, and other factors well known in the medical field. The daily dose may be in the range of 0.01 $\mu$g/kg to 10 g/kg, or in the range of 0.01 mg/kg to 1 g/kg. Administration may be performed once a day or several times a day. Such dosages should not be construed as limiting the scope of the present invention in any aspect.

In still yet another aspect of the present invention, there is provided a use of the fusion protein for treating or preventing cancer. Here, the fusion protein, the cancer, the treatment, and the prevention are as described above.

In still yet another aspect of the present invention, there is provided a method for treating or preventing cancer, the method including administering the fusion protein to a subject. Here, the fusion protein, the administration, the cancer, the treatment, and the prevention are as described above. The subject may be a mammal and preferably a human. In addition, the subject may be a patient suffering from cancer, or a subject who is more likely to suffer from cancer.

Route of administration, dose, and frequency of administration of the fusion protein or fusion protein dimer may vary depending on the patient's condition and the presence or absence of side effects, and thus the fusion protein or fusion protein dimer may be administered to a subject in various ways and amounts. The optimal administration method, dose, and frequency of administration can be selected in an appropriate range by those skilled in the art. In addition, the fusion protein or fusion protein dimer may be administered in combination with other drugs or physiologically active substances whose therapeutic effect is known with respect to a disease to be treated, or may be formulated in the form of combination preparations with other drugs.

**Mode for Carrying out the Invention**

Hereinafter, the present invention will be described in more detail by way of the following examples. These examples are only for illustrating the present invention, and it will be apparent to one of ordinary skill in the art that the scope of the present invention is not to be construed as being limited by these examples.

**I. Preparation of fusion protein**

**Preparation Example 1. Preparation of anti-hLAG-3 antibody-hIgG4 Fc-hIL-2v2 fusion protein: GI-104E1**

In order to produce a fusion protein comprising an anti-hLAG-3 antibody and hIL-2v2, a polynucleotide was inserted into pcDNA3_4 vector (Invitrogen) through the Expression and Optimization service of GenScript. Specifically, the polynucleotide (SEQ ID NO: 18) contains a nucleotide sequence (SEQ ID NO: 16) which encodes a fusion protein that contains anti-hLAG-3VL (anti-hLAG-3(1E09)VL-kappa+CL), in this order, from the N-terminus; and a polynucleotide (SEQ ID NO: 17) contains a nucleotide sequence (SEQ ID NO: 27) which encodes a fusion protein that contains anti-hLAG-3VH (anti-hLAG-3(1E09)VH+CH1), an Fc domain (F02(hIgG4Fc)), a linker (G4S linker), and human IL-2v2, in this order, from the N-terminus. The vector was introduced into CHO cells (HD CHO-S). After the vector was introduced, culture was performed for 14 days in a serum-free HD CHO-S™ expression medium of 37°C, and 8% $CO_2$ concentration.

Then, the culture was harvested and the fusion protein was purified using affinity chromatography (affinity purification column) containing MabSelect SuRe™LX.

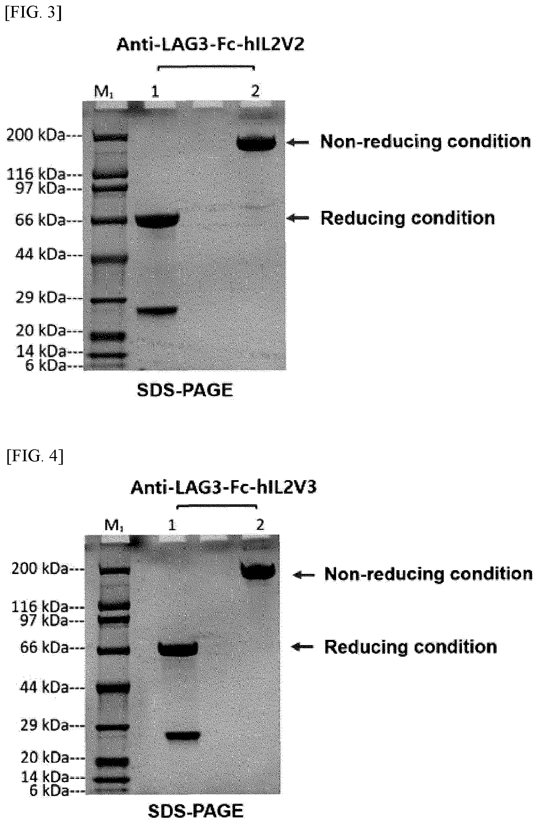

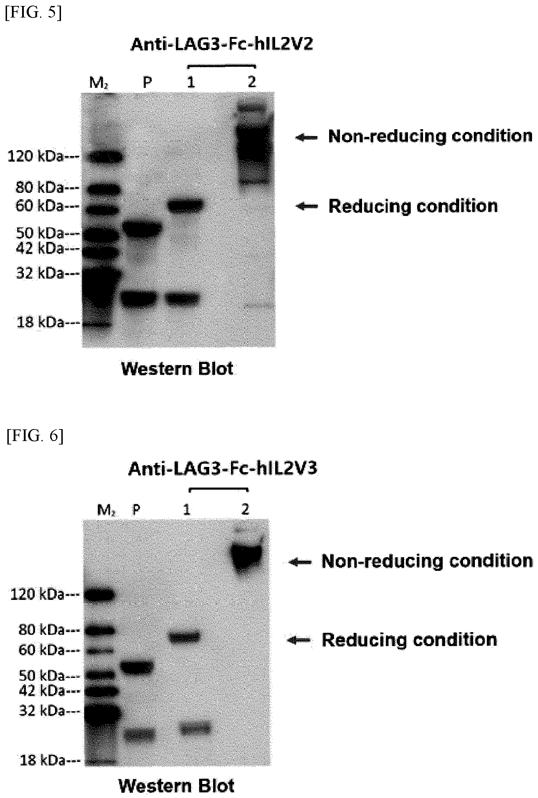

The isolated and purified fusion protein was subjected to SDS-PAGE and western blot under reducing (R) or non-reducing (NR) conditions, and confirmed through HPLC analysis to check its molecular weight and purity (FIGS. 3 and 5). The concentration thereof was measured through Bradford assay, and it was identified that the fusion protein was contained at a concentration of 0.69 mg/ml.

**Preparation Example 2. Preparation of control antibody (anti-hLAG-3 antibody and α-LAG-3)**

In order to produce an anti-hLAG-3 antibody as a control, a polynucleotide was inserted into pcDNA3_4 vector (Invitrogen) through the Expression and Optimization service of GenScript. Specifically, the polynucleotide (SEQ ID NO: 18) contains a nucleotide sequence (SEQ ID NO: 16) which encodes a fusion protein that contains anti-hLAG-3VL (anti-hLAG-3(1E09)VL-kappa+CL), in this order, from the N-terminus; and a polynucleotide (SEQ ID NO: 29) contains a nucleotide sequence (SEQ ID NO: 15) which encodes a fusion protein that contains anti-hLAG-3VH (anti-hLAG-3(1E09)VH+CH1) and an Fc domain (F02(hIgG4Fc)), in this order, from the N-terminus. The vector was introduced into CHO cells (HD CHO-S). After the vector was introduced, culture was performed for 14 days in a serum-free HD CHO-S™ expression medium of 37°C, and 8% $CO_2$ concentration. Then, the culture was harvested and the fusion protein was purified using affinity chromatography (affinity purification column) containing MabSelect SURE™LX.

**Preparation Example 3. Preparation of hIgG4 Fc-hIL-2v2: Fc-IL-2v2**

In order to produce a fusion protein dimer comprising an Fc domain and an IL-2 variant, a polynucleotide was synthesized through the Invitrogen GeneArt Gene Synthesis service of ThermoFisher Scientific. Specifically, the polynucleotide (SEQ ID NO: 25) contains a nucleotide sequence (SEQ ID NO: 11) which encodes a fusion protein that contains an Fc domain (SEQ ID NO: 14), a linker (SEQ ID NO: 19), and an IL-2 variant (2M) (R38A and F42A) (SEQ ID NO: 20) having two amino acid substitutions, in this order, from the N-terminus. The polynucleotide was inserted into pcDNA3_4 vector (Invitrogen). In addition, the vector was introduced into CHO cells (EXPI-CHO™) to express the fusion protein of SEQ ID NO: 11. After the vector was introduced, culture was performed for 7 days in an environment of 37°C, 125 RPM, and 8% $CO_2$ concentration. Then, the culture was harvested and the fusion protein was purified therefrom.

**Preparation Example 4. Preparation of hIgG4 Fc-hIL-2v3: Fc-IL-2v3**

In order to produce a fusion protein comprising an Fc domain and an IL-2 variant, a polynucleotide was synthesized through the Invitrogen GeneArt Gene Synthesis service of ThermoFisher Scientific. Specifically, the polynucleotide (SEQ ID NO: 26) contains a nucleotide sequence (SEQ ID NO: 24) which encodes a fusion protein that contains an Fc domain (SEQ ID NO: 14), a linker (SEQ ID NO: 19), and an IL-2 variant (3M) (R38A, F42A, and E61R) (SEQ ID NO: 21) having three amino acid substitutions, in this order, from the N-terminus. The polynucleotide was inserted into pcDNA3_4 vector (Invitrogen). In addition, the vector was introduced into CHO cells (Expi-CHO™) to express the fusion protein of SEQ ID NO: 24. After the vector was introduced culture was performed for 7 days in an environment of 37°C, 125 RPM, and 8% $CO_2$ concentration. Then, the culture was harvested and the fusion protein was purified therefrom.

**Preparation Example 5. Preparation of anti-hLAG-3 antibody-hIgG4 Fc-hIL-2v3 fusion protein: GI-104E2**

In order to produce a fusion protein comprising an anti-LAG-3 antibody and hIL-2v3, a polynucleotide was inserted into pcDNA3_4 vector (Invitrogen) through the Expression and Optimization service of GenScript. Specifically, the polynucleotide (SEQ ID NO: 18) contains a nucleotide sequence (SEQ ID NO: 16) which encodes a fusion protein that contains anti-hLAG-3VL (anti-hLAG-3(1E09)VL-kappa+CL) in this order from the N-terminus; and a polynucleotide (SEQ ID NO: 23) contains a nucleotide sequence (SEQ ID NO: 28) which encodes a fusion protein that contains anti-hLAG-3VH (anti-hLAG-3(1E09)VH+CH1), an Fc domain (F02(hIgG4Fc)), a linker (G4S linker), and human IL-2v3, in this order, from the N-terminus. The vector was introduced into CHO cells (HD CHO-S). After the vector was introduced, culture was performed for 14 days in a serum-free HD CHO-S™ expression medium of 37°C, and 8% $CO_2$ concentration. Then, the culture was harvested and the fusion protein was purified using affinity chromatography (affinity purification column) containing MabSelect SURE™LX.

The isolated and purified fusion protein was subjected to SDS-PAGE and western blot under reducing (R) or non-reducing (NR) conditions, and confirmed through HPLC analysis to check its molecular weight and purity (FIGS. 4 and 6). The concentration thereof was measured through Bradford assay, and it was identified that the fusion protein was contained at a concentration of 0.51 mg/ml.

**II. Identification of immune activity of fusion protein**

**Experimental Example 1. Identification of function of T cell activation of fusion protein by LAG-3 blockade assay**

**Experimental Example 1.1. Identification of effect of GI-104E1 on function of T cells**

LAG-3 is an immune checkpoint inhibitor expressed in T cells and NK cells, and has a structure similar to that of CD4. However, LAG-3 has 30 additional amino acids in a D1 domain and thus has a high affinity with MHC class II. Due to such structural features, LAG-3 is known to inhibit T cell activation.

This experiment attempted to evaluate the T cell activation function for the fusion protein using the LAG-3 blockade bioassay system (Promega, JA1115). Specifically, one vial of MHCII APC cells kept in liquid nitrogen were thawed in a 37°C constant temperature bath for 2 minutes, and then added to 14.5 ml of a cell recovery medium (90% DMEM + 10% FBS) containing a TCR activating antigen, and the resultant was mixed well. An MHCII APC suspension was added to each well of a 96-well white cell culture plate (Corning cat no. 3917) at 100 μl per well, and cultured in an incubator at 37°C and 5% $CO_2$ for 24 hours.

The 96-well white cell culture plate containing the MHCII APC cells cultured for 24 hours was taken out and the culture medium in the plate was removed. Then, 40 μl of GI-104E1 and 1E09 (Relatlimab surrogates) as a positive control were treated per well at various concentrations, and 40 μl of an assay buffer as a negative control was added. Then, the white cell culture plate was covered and placed at room temperature until the LAG-3 effector cells were prepared.

One vial of the LAG-3 effector cells kept in liquid nitrogen was thawed in a 37°C constant temperature bath for 2 minutes, and then added to 7 ml of an assay buffer (90% RPMI + 10% FBS), and the resultant was mixed well. 40 μl of the mixed suspension was added to each well of the 96-well white cell culture plate stored at room temperature, and reacted in an incubator at 37°C and 5% $CO_2$ for 5 hours. After the reaction was completed, the resultant was allowed to stand at room temperature for 10 minutes, and then 80 μl of a Bio-Glo reagent was added to each well while taking care to avoid bubbles. The Bio-Glo reagent was also added to two of the outermost wells and the wells were used as blanks to correct the background signal. The reaction was allowed to proceed at room temperature for 10 minutes, and then luminescence was measured using the GloMax® Discover System (Promega, USA).

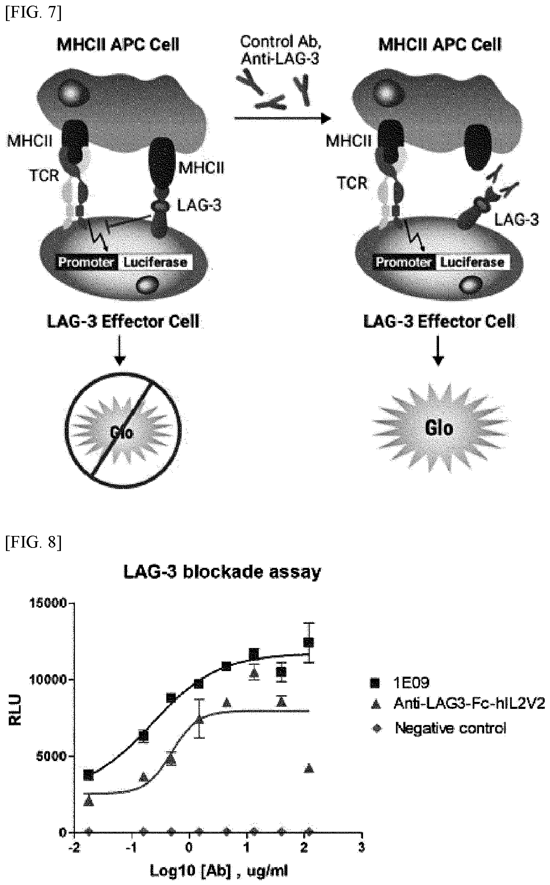

As a result, it was confirmed that GI-104E1 could activate the T cell function by binding to LAG-3 expressed in effector T cells and inhibiting the function of the LAG-3 (FIG. 8).

**Experimental Example 1.2. Identification of effect of GI-104E2 on T cell function**

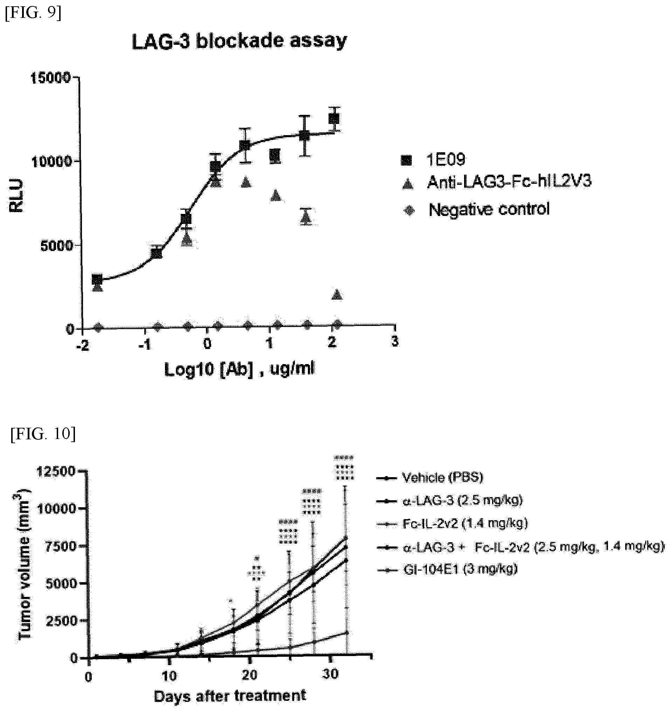

As a result of performing LAG-3 blockade assay on GI-104E2 in the same manner as in Experimental Example 1.1, it was confirmed that GI-104E2 could activate the T cell function by binding to LAG-3 expressed in effector T cells and inhibiting the function of the LAG-3 (FIG. 9).

**Experimental Example 2. Identification of anticancer effect of fusion protein**

**Experimental Example 2.1. Identification of anticancer effect of GI-104E1 in mouse-derived colorectal cancer cell transplanted mice**

BALB/c mice (female, 7-week-old) acquired from Orient Bio Inc. were subjected to an acclimation period of 7 days. Then, $5 \times 10^6$ cells of CT26 cancer cell line (ATCC, U.S.A.) were diluted with 1 ml of PBS, and allotransplantation of the resultant was performed by subcutaneous administration at 100 μl per subject in the right dorsal region of the mice. A certain period of time after the cancer cell transplantation, the tumor volume was measured and subjects that reached about 50 mm$^3$ to 120 mm$^3$ were selected, and then the selected mice were grouped evenly based on tumor size and body weight, each group containing 8 mice. Thereafter, test groups were formed as shown in Table 3, and test substances were administered intraperitoneally. A total of three administrations were given once a week after the first administration. Regarding the size of the transplanted tumor, the tumor volumes in all cases were measured twice a week for 4 weeks.

[Table 3]

| Group | Drug | Dosage (mg/kg) | Dosage (mL/kg) | Administration route | Administration frequency | Number of subjects |
|-------|------|----------------|----------------|---------------------|--------------------------|--------------------|
| G1 | Vehicle (PBS) | - | 5 | Intraperitoneal | Once a week, for 3 weeks | 8 |

(continued)

| Group | Drug | Dosage (mg/kg) | Dosage (mL/kg) | Administration route | Administration frequency | Number of subjects |
|-------|------|----------------|----------------|----------------------|--------------------------|--------------------|
| G2 | Anti-LAG-3 antibody | 2.5 | 5 | Intraperitoneal | Once a week, for 3 weeks | 8 |
| G3 | Fc-IL-2v2 | 1.4 | 5 | Intraperitoneal | Once a week, for 3 weeks | 8 |
| G4 | Anti-LAG-3 antibody + Fc-IL-2v2 | 2.5 + 1.4 | 5 | Intraperitoneal | Once a week, for 3 weeks | 8 |
| G5 | GI-104E1 | 3 | 5 | Intraperitoneal | Once a week, for 3 weeks | 8 |

The measurement results were shown as a graph using the mean and standard deviation (SD) values of each group. Moreover, the statistical significance of the decrease in the tumor volume compared to the vehicle (#$p \leq 0.05$, ####$p \leq 0.0001$), and the statistical significance of the difference in tumor volumes compared to the GI-104E1 administration group (*$p <\_ 0.05$, **$p \leq 0.01$, ***$p \leq 0.001$, ****$p \leq 0.0001$) were analyzed using two-way analysis of variance and a Dunnett T3 test.

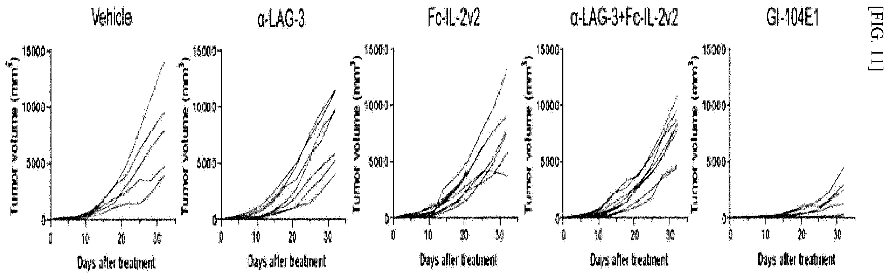

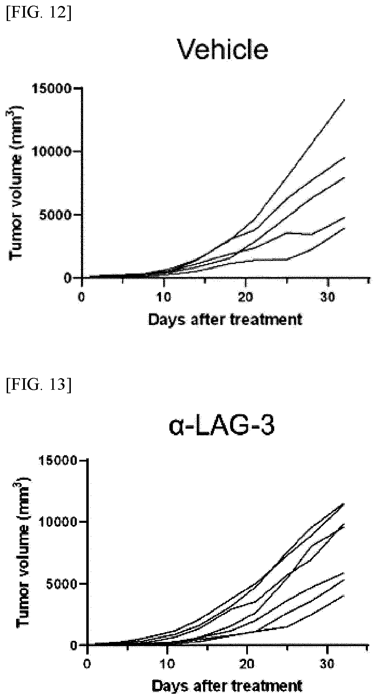

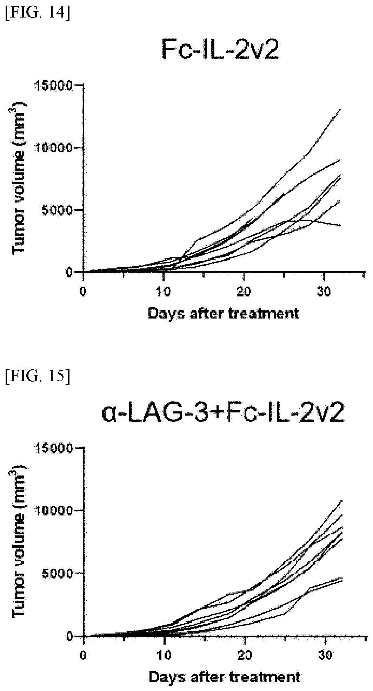

As a result, in the CT26 cancer cell line transplanted mice, the group in which GI-104E1 was administered in a dose of 3 mg/kg showed the significant inhibition of the tumor growth at the midpoint of observation and at the end of the experiment, compared to the negative control (#$p \leq 0.05$, ####$p \leq 0.0001$, two-way analysis of variance (ANOVA)). In addition, even when compared to the administration of the anti-LAG-3 antibody or the Fc-IL-2v2 with the molar number equivalent to that of GI-104E1, the GI-104E1 administration significantly inhibited the growth of tumors. In particular, GI-104E1 showed a significant tumor inhibitory effect at the midpoint of observation and at the end of the experiment, even compared to the experimental group in which the anti-LAG-3 antibody and the Fc-IL-2v2 were co-administered (*$p < 0.05$, **$p \leq 0.01$, ***$p <\_ 0.001$, ****$p \leq 0.0001$, two-way analysis of variance (ANOVA)) (FIGS. 10 to 16).

When the change in the tumor growth rate was observed at the end of the test, the number of subjects showing a decrease in the tumor growth rate of 50% or greater was 1 in the negative control, 1 in the group in which the anti-LAG-3 antibody was administered, 0 in the group in which the Fc-IL-2v2 was administered, and 0 in the group in which the anti-LAG-3 antibody and the Fc-IL-2v2 were co-administered. Meanwhile, in the case of the experimental group in which GI-104E1 was administered, it was confirmed that a decrease in the tumor growth rate of 50% or greater was observed in seven subjects, and the number of subjects showing a decrease in the tumor growth rate of 80% or greater was 5 (FIG. 17).

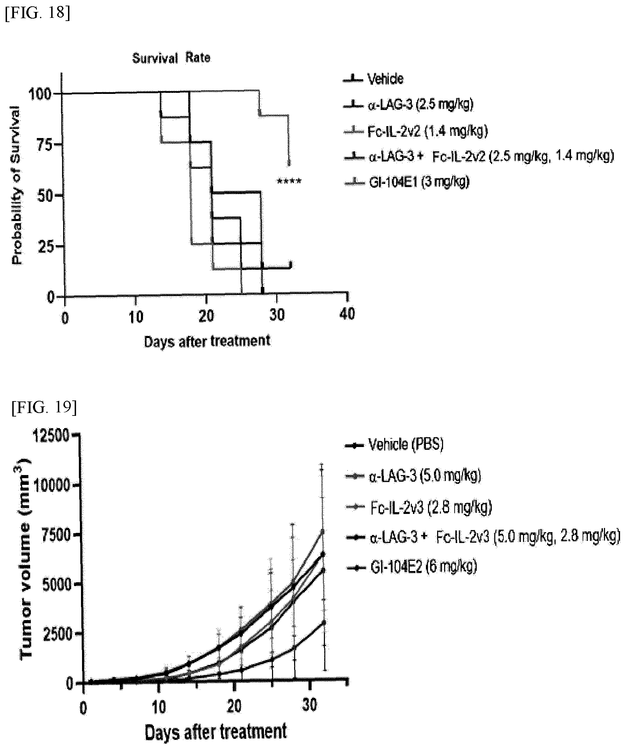

Furthermore, the survival rates of the experimental group in which GI-104E1 was administered and the experimental groups in which the other experimental controls and the negative control were administered were analyzed on the basis of the death of the experimental subjects or the generation of the tumor volume of 2,000 mm$^3$ in the subjects, and the statistical significance of the difference in survival rates among the experimental groups was analyzed by the Mantel-Cox test. As a result, it was confirmed that the experimental group in which GI-104E1 was administered showed a higher survival rate compared to the experimental groups in which the other experimental controls and the negative control were administered, and such a difference in survival rates among the experimental groups was statistically significant (****$p \leq 0.0001$) (FIG. 18).

**Experimental Example 2.2. Identification of anticancer effect of GI-104E2 in mouse-derived colorectal cancer cell tranplanted mice**

BALB/c mice (female, 7-week-old) acquired from Orient Bio Inc. were subjected to an acclimation period of 7 days. $5 \times 10^6$ cells of CT26 cancer cell line (ATCC, U.S.A.) were diluted with 1 ml of PBS, and allotransplantation of the resultant was performed by subcutaneous administration at 100 μl per subject in the right dorsal region of the mice. A certain period of time after the cancer cell transplantation, the tumor volume was measured and subjects that reached about 50 mm$^3$ to 120 mm$^3$ were selected, and then the selected mice were grouped evenly based on tumor size and body weight, each group containing 3 mice. Thereafter, test groups were formed as shown in Table 4, and test substances were administered intraperitoneally. A total of three administrations were given once a week after the first administration. Regarding the size of the transplanted tumor, the tumor volumes in all cases were measured twice a week for 4 weeks.

[Table 4]

| Group | Drug | Dosage (mg/kg) | Dosage (mL/kg) | Administration route | Administration frequency | Number of subjects |
|---|---|---|---|---|---|---|
| G1 | Vehicle (PBS) | - | 5 | Intraperitoneal | Once a week, for 3 weeks | 3 |
| G2 | Anti-LAG-3 antibody | 5 | 5 | Intraperitoneal | Once a week, for 3 weeks | 3 |
| G3 | Fc-IL-2v3 | 2.8 | 5 | Intraperitoneal | Once a week, for 3 weeks | 3 |
| G4 | Anti-LAG-3 antibody + Fc-IL-2v3 | 5 + 2.8 | 5 | Intraperitoneal | Once a week, for 3 weeks | 3 |
| G5 | GI-104E2 | 6 | 5 | Intraperitoneal | Once a week, for 3 weeks | 3 |

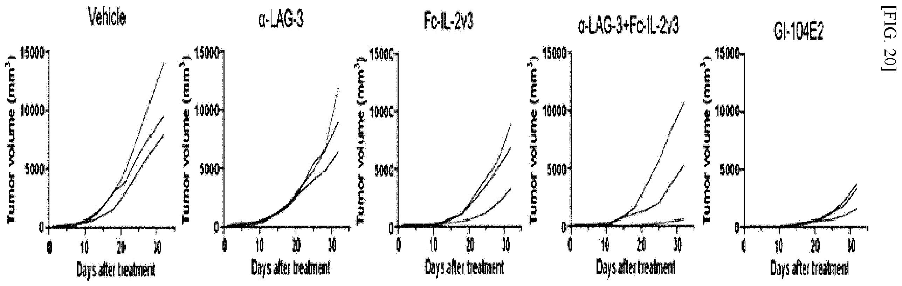

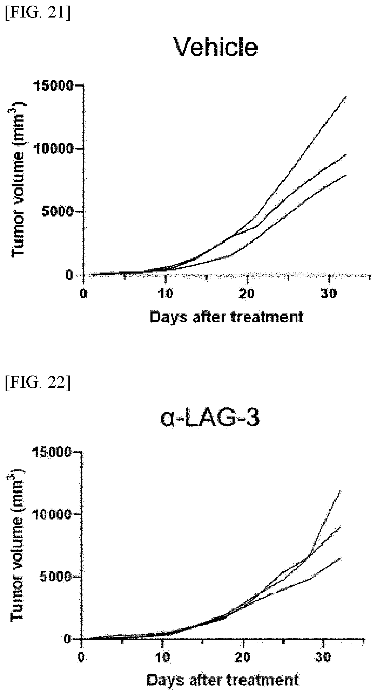

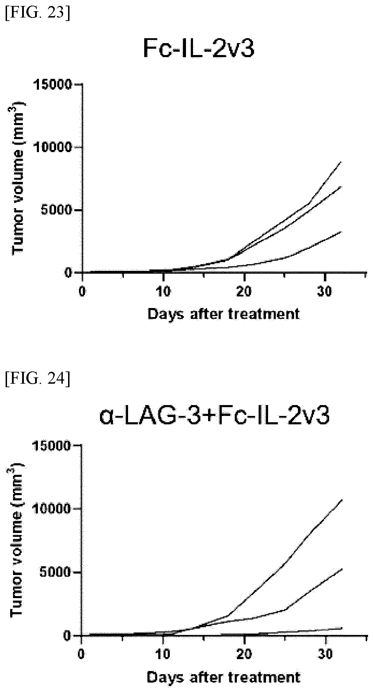

As a result, in the CT26 cancer cell line transplanted mice, the group in which GI-104E2 was administered in a dose of 6 mg/kg showed the inhibition of the tumor growth at the midpoint of observation and at the end of the experiment, compared to the negative control. In addition, even when compared to the administration of the anti-LAG-3 antibody or the Fc-IL-2v3 with the molar number equivalent to that of 6 mg/kg of the GI-104E2, the GI-104E2 administration group showed an excellent tumor growth inhibitory effect. In particular, GI-104E2 showed an excellent tumor inhibitory effect at the midpoint of observation and at the end of the experiment, even compared to the experimental group in which the anti-LAG-3 antibody and the Fc-IL-2v3 were co-administered (FIGS. 19 to 25).

Furthermore, when the change in the tumor growth rate was observed at the end of the test, the number of subjects showing a decrease in the tumor growth rate of 50% or greater was 1 in the negative control, 0 in the group in which the anti-LAG-3 antibody was administered, 1 in the group in which the Fc-IL-2v3 was administered, and 1 in the group in which the anti-LAG-3 antibody and the Fc-IL-2v3 were co-administered. Meanwhile, in the case of the experimental group in which GI-104E2 was administered, it was confirmed that a decrease in the tumor growth rate of 50% or greater was observed in all subjects and the number of subjects showing a decrease in the tumor growth rate of 80% or greater was 1 (FIG. 26).

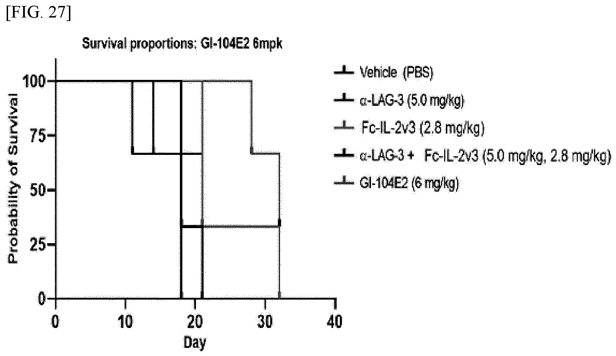

The survival rates of the experimental group in which GI-104E2 was administered and the experimental groups in which the other experimental controls and the negative control were administered were analyzed on the basis of the death of the experimental subjects or the generation of the tumor volume of 2,000 $mm^3$ in the subjects. As a result, it was confirmed that the experimental group in which GI-104E2 was administered showed a higher survival rate compared to the experimental groups in which the other experimental controls and the negative control were administered (FIG. 27).

## Claims

1.   A fusion protein comprising:

   an antibody that specifically binds to LAG-3; and
   an IL-2 protein.

2.   The fusion protein of claim 1, wherein the antibody that specifically binds to LAG-3 comprises

   a heavy chain variable region comprising HCDR1 of SEQ ID NO: 1, HCDR2 of SEQ ID NO: 2, and HCDR3 of SEQ ID NO: 3; and
   a light chain variable region comprising LCDR1 of SEQ ID NO: 5, LCDR2 of SEQ ID NO: 6, and LCDR3 of SEQ ID NO: 7.

3.   The fusion protein of claim 1, wherein the antibody that specifically binds to LAG-3 comprises
   a heavy chain variable region comprising the amino acid sequence of SEQ ID NO: 4, and a light chain variable region comprising the amino acid sequence of SEQ ID NO: 8.

4.   The fusion protein of claim 1, wherein the fusion protein comprises two IL-2 proteins.

**5.** The fusion protein of claim 1, wherein the IL-2 protein is an IL-2 variant.

**6.** The fusion protein of claim 5, wherein the IL-2 variant is substituted at a position selected from the group consisting of R38A, F42A, Y45A, E61R, L72G, and combinations thereof.

**7.** The fusion protein of claim 5, wherein the IL-2 variant has the amino acid sequence of SEQ ID NO: 20 or SEQ ID NO: 21.

**8.** The fusion protein of claim 1, wherein the antibody that specifically binds to LAG-3 and the IL-2 protein are attached to each other via a linker.

**9.** The fusion protein of claim 8, wherein the linker is a peptide linker.

**10.** A polynucleotide encoding the fusion protein of any one of claims 1 to 9.

**11.** An expression vector comprising the polynucleotide of claim 10.

**12.** A transformed cell into which the expression vector of claim 11 has been introduced.

**13.** A method for preparing a fusion protein, comprising:

    culturing the transformed cell of claim 12; and
    collecting a fusion protein from a culture medium.

**14.** A pharmaceutical composition for treating or preventing cancer, comprising the fusion protein of any one of claims 1 to 9.

**15.** The pharmaceutical composition of claim 14, wherein the cancer is one selected from the group consisting of gastric cancer, liver cancer, lung cancer, colorectal cancer, breast cancer, prostate cancer, ovarian cancer, pancreatic cancer, cervical cancer, thyroid cancer, laryngeal cancer, acute myeloid leukemia, brain tumor, neuroblastoma, retinoblastoma, head and neck cancer, salivary gland cancer, and lymphoma.

**16.** Use of the fusion protein of any one of claims 1 to 9 for treating or preventing cancer.

**17.** Use of the fusion protein of any one of claims 1 to 9 for the manufacture of a medicament for treatment or prevention of cancer.

**18.** A method for treating or preventing cancer, comprising administering the fusion protein of any one of claims 1 to 9 to a subject.

[FIG. 1]

Anti-hLag3 LC (kappa)

Anti-hLag3 hIgG4Fc-HC

hIL2v2

[FIG. 2]

Anti-hLag3 LC (kappa)

Anti-hLag3 hIgG4Fc-HC

hIL2v3

[FIG. 3]

## Anti-LAG3-Fc-hIL2V2

←  Non-reducing condition

←  Reducing condition

SDS-PAGE

[FIG. 4]

## Anti-LAG3-Fc-hIL2V3

←  Non-reducing condition

←  Reducing condition

SDS-PAGE

[FIG. 5]

**Anti-LAG3-Fc-hIL2V2**

Western Blot

[FIG. 6]

**Anti-LAG3-Fc-hIL2V3**

Western Blot

[FIG. 7]

[FIG. 8]

[FIG. 9]

**LAG-3 blockade assay**

- ■ 1E09
- ▲ Anti-LAG3-Fc-hIL2V3
- ◆ Negative control

[FIG. 10]

- Vehicle (PBS)
- α-LAG-3 (2.5 mg/kg)
- Fc-IL-2v2 (1.4 mg/kg)
- α-LAG-3 + Fc-IL-2v2 (2.5 mg/kg, 1.4 mg/kg)
- GI-104E1 (3 mg/kg)

[FIG. 11]

[FIG. 12]

**Vehicle**

[FIG. 13]

**α-LAG-3**

[FIG. 14]

## Fc-IL-2v2

[FIG. 15]

## α-LAG-3+Fc-IL-2v2

[FIG. 16]

GI-104E1

[FIG. 17]

[FIG. 18]

Survival Rate

Legend:
- Vehicle
- α-LAG-3 (2.5 mg/kg)
- Fc-IL-2v2 (1.4 mg/kg)
- α-LAG-3 + Fc-IL-2v2 (2.5 mg/kg, 1.4 mg/kg)
- GI-104E1 (3 mg/kg)

****

[FIG. 19]

Legend:
- Vehicle (PBS)
- α-LAG-3 (5.0 mg/kg)
- Fc-IL-2v3 (2.8 mg/kg)
- α-LAG-3 + Fc-IL-2v3 (5.0 mg/kg, 2.8 mg/kg)
- GI-104E2 (6 mg/kg)

[FIG. 20]

[FIG. 21]

Vehicle

[FIG. 22]

α-LAG-3

[FIG. 23]

Fc-IL-2v3

[FIG. 24]

α-LAG-3+Fc-IL-2v3

[FIG. 25]

[FIG. 26]

[FIG. 27]

Survival proportions: GI-104E2 6mpk

━┴━ Vehicle (PBS)

━┴━ α-LAG-3 (5.0 mg/kg)

━┴━ Fc-IL-2v3 (2.8 mg/kg)

━┴━ α-LAG-3 +   Fc-IL-2v3 (5.0 mg/kg, 2.8 mg/kg)

━┴━ GI-104E2 (6 mg/kg)

# EP 4 174 088 A1

## INTERNATIONAL SEARCH REPORT

| International application No. |
|---|
| **PCT/KR2021/008198** |

| **A.** | **CLASSIFICATION OF SUBJECT MATTER** |
|---|---|
| | **C07K 16/28**(2006.01)i; **C07K 14/55**(2006.01)i; **A61K 38/00**(2006.01)i; **A61P 35/00**(2006.01)i; **A61K 39/00**(2006.01)i |

According to International Patent Classification (IPC) or to both national classification and IPC

| **B.** | **FIELDS SEARCHED** |
|---|---|

Minimum documentation searched (classification system followed by classification symbols)

C07K 16/28(2006.01); A61K 38/20(2006.01); A61K 39/00(2006.01); C07K 14/55(2006.01); C07K 14/715(2006.01); C07K 19/00(2006.01)

Documentation searched other than minimum documentation to the extent that such documents are included in the fields searched

Korean utility models and applications for utility models: IPC as above
Japanese utility models and applications for utility models: IPC as above

Electronic data base consulted during the international search (name of data base and, where practicable, search terms used)

eKOMPASS (KIPO internal) & keywords: LAG-3, IL-2, 항체(antibody), 벡터(vector), 링커(linker)

| **C.** | **DOCUMENTS CONSIDERED TO BE RELEVANT** |
|---|---|

| Category* | Citation of document, with indication, where appropriate, of the relevant passages | Relevant to claim No. |
|---|---|---|
| A | KR 10-2019-0032355 A (KYMAB LIMITED) 27 March 2019 (2019-03-27)<br>    See abstract; claims 1-64; and paragraph [0003]. | 1-17 |
| A | KRAMAN, M. et al. FS118, a Bispecific Antibody Targeting LAG-3 and PD-L1, Enhances T-Cell Activation Resulting in Potent Antitumor Activity. CLINICAL CANCER RESEARCH. 16 April 2020, vol. 26, pp. 3333-3344.<br>    See entire document. | 1-17 |
| A | KR 10-2020-0002678 A (Y-BIOLOGICS INC.) 08 January 2020 (2020-01-08)<br>    See entire document. | 1-17 |
| A | WO 2019-191295 A1 (BRISTOL-MYERS SQUIBB COMPANY) 03 October 2019 (2019-10-03)<br>    See entire document. | 1-17 |
| A | WO 2020-113403 A1 (BEIJING PERCANS ONCOLOGY CO., LTD.) 11 June 2020 (2020-06-11)<br>    See entire document. | 1-17 |

| ☐ Further documents are listed in the continuation of Box C. | ☑ See patent family annex. |
|---|---|

| * | Special categories of cited documents: | "T" | later document published after the international filing date or priority date and not in conflict with the application but cited to understand the principle or theory underlying the invention |
|---|---|---|---|
| "A" | document defining the general state of the art which is not considered to be of particular relevance | | |
| "D" | document cited by the applicant in the international application | "X" | document of particular relevance; the claimed invention cannot be considered novel or cannot be considered to involve an inventive step when the document is taken alone |
| "E" | earlier application or patent but published on or after the international filing date | | |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | "&" | document member of the same patent family |
| "P" | document published prior to the international filing date but later than the priority date claimed | | |

| Date of the actual completion of the international search | Date of mailing of the international search report |
|---|---|
| **13 October 2021** | **13 October 2021** |

| Name and mailing address of the ISA/KR | Authorized officer |
|---|---|
| **Korean Intellectual Property Office**<br>**Government Complex-Daejeon Building 4, 189 Cheongsa-ro, Seo-gu, Daejeon 35208** | |
| Facsimile No. **+82-42-481-8578** | Telephone No. |

Form PCT/ISA/210 (second sheet) (July 2019)

## INTERNATIONAL SEARCH REPORT

International application No.

**PCT/KR2021/008198**

| Box No. I | Nucleotide and/or amino acid sequence(s) (Continuation of item 1.c of the first sheet) |
|---|---|

1. With regard to any nucleotide and/or amino acid sequence disclosed in the international application, the international search was carried out on the basis of a sequence listing:

    a. ☑ forming part of the international application as filed:

        ☑ in the form of an Annex C/ST.25 text file.

        ☐ on paper or in the form of an image file.

    b. ☐ furnished together with the international application under PCT Rule 13*ter*.1(a) for the purposes of international search only in the form of an Annex C/ST.25 text file.

    c. ☐ furnished subsequent to the international filing date for the purposes of international search only:

        ☐ in the form of an Annex C/ST.25 text file (Rule 13*ter*.1(a)).

        ☐ on paper or in the form of an image file (Rule 13*ter*.1(b) and Administrative Instructions, Section 713).

2. ☐ In addition, in the case that more than one version or copy of a sequence listing has been filed or furnished, the required statements that the information in the subsequent or additional copies is identical to that forming part of the application as filed or does not go beyond the application as filed, as appropriate, were furnished.

3. Additional comments:

Form PCT/ISA/210 (continuation of first sheet) (July 2019)

## INTERNATIONAL SEARCH REPORT

| International application No. |
| --- |
| **PCT/KR2021/008198** |

**Box No. II      Observations where certain claims were found unsearchable (Continuation of item 2 of first sheet)**

This international search report has not been established in respect of certain claims under Article 17(2)(a) for the following reasons:

1. ☑  Claims Nos.: **18**
   because they relate to subject matter not required to be searched by this Authority, namely:

   Claim 18 pertains to a method for treatment of the human body by surgery or therapy, as well as a diagnostic method (PCT Article 17(2)(a)(i) and PCT Rule 39.1(iv)).

2. ☐  Claims Nos.:
   because they relate to parts of the international application that do not comply with the prescribed requirements to such an extent that no meaningful international search can be carried out, specifically:

3. ☐  Claims Nos.:
   because they are dependent claims and are not drafted in accordance with the second and third sentences of Rule 6.4(a).

Form PCT/ISA/210 (continuation of first sheet) (July 2019)

## INTERNATIONAL SEARCH REPORT
### Information on patent family members

International application No.

**PCT/KR2021/008198**

| Patent document cited in search report | | Publication date (day/month/year) | Patent family member(s) | | | Publication date (day/month/year) |
|---|---|---|---|---|---|---|
| KR 10-2019-0032355 | A | 27 March 2019 | AU | 2017-281830 | A1 | 13 December 2018 |
| | | | BR | 112018076260 | A2 | 26 March 2019 |
| | | | BR | 112018076281 | A2 | 26 March 2019 |
| | | | BR | 112019002529 | A2 | 28 May 2019 |
| | | | CA | 3026474 | A1 | 28 December 2017 |
| | | | CA | 3026477 | A1 | 28 December 2017 |
| | | | CA | 3032897 | A1 | 15 February 2018 |
| | | | CN | 109475602 | A | 15 March 2019 |
| | | | CN | 109475603 | A | 15 March 2019 |
| | | | CN | 109689688 | A | 26 April 2019 |
| | | | EP | 3292967 | A1 | 14 March 2018 |
| | | | EP | 3292967 | B1 | 28 October 2020 |
| | | | EP | 3471753 | A1 | 24 April 2019 |
| | | | EP | 3471754 | A1 | 24 April 2019 |
| | | | EP | 3497128 | A2 | 19 June 2019 |
| | | | EP | 3559041 | A1 | 30 October 2019 |
| | | | JP | 2019-528083 | A | 10 October 2019 |
| | | | JP | 2019-534892 | A | 05 December 2019 |
| | | | JP | 2020-502198 | A | 23 January 2020 |
| | | | JP | 2020-511932 | A | 23 April 2020 |
| | | | KR | 10-2019-0030208 | A | 21 March 2019 |
| | | | KR | 10-2019-0038618 | A | 08 April 2019 |
| | | | MX | 2018016364 | A | 28 November 2019 |
| | | | RU | 2018147413 | A | 21 July 2020 |
| | | | RU | 2018147423 | A | 21 July 2020 |
| | | | RU | 2018147423 | A3 | 16 October 2020 |
| | | | RU | 2019104980 | A | 11 September 2020 |
| | | | RU | 2019104980 | A3 | 22 October 2020 |
| | | | SG | 11201810509 | A | 28 December 2018 |
| | | | TW | 201803904 | A | 01 February 2018 |
| | | | TW | 201803905 | A | 01 February 2018 |
| | | | TW | 201811820 | A | 01 April 2018 |
| | | | TW | 201811826 | A | 01 April 2018 |
| | | | TW | 201900680 | A | 01 January 2019 |
| | | | TW | I640536 | B | 11 November 2018 |
| | | | US | 10604576 | B2 | 31 March 2020 |
| | | | US | 2017-0362321 | A1 | 21 December 2017 |
| | | | US | 2018-0066058 | A1 | 08 March 2018 |
| | | | US | 2019-0202917 | A1 | 04 July 2019 |
| | | | US | 2019-0330345 | A1 | 31 October 2019 |
| | | | US | 2019-0330351 | A1 | 31 October 2019 |
| | | | US | 2019-0338032 | A1 | 07 November 2019 |
| | | | US | 2020-0190191 | A1 | 18 June 2020 |
| | | | US | 9567399 | B1 | 14 February 2017 |
| | | | US | 9617338 | B1 | 11 April 2017 |
| | | | US | 9957323 | B2 | 01 May 2018 |
| | | | WO | 2017-220988 | A1 | 28 December 2017 |
| | | | WO | 2017-220989 | A1 | 28 December 2017 |
| | | | WO | 2017-220990 | A1 | 28 December 2017 |
| | | | WO | 2018-029474 | A2 | 15 February 2018 |

Form PCT/ISA/210 (patent family annex) (July 2019)

**INTERNATIONAL SEARCH REPORT**
Information on patent family members

International application No.

**PCT/KR2021/008198**

| Patent document cited in search report | | | Publication date (day/month/year) | Patent family member(s) | | | Publication date (day/month/year) |
|---|---|---|---|---|---|---|---|
| | | | | WO | 2018-029474 | A3 | 22 March 2018 |
| | | | | WO | 2018-115859 | A1 | 28 June 2018 |
| KR | 10-2020-0002678 | A | 08 January 2020 | CN | 112119094 | A | 22 December 2020 |
| | | | | EP | 3816187 | A1 | 05 May 2021 |
| | | | | KR | 10-2250234 | B1 | 10 May 2021 |
| | | | | US | 2021-0238283 | A1 | 05 August 2021 |
| | | | | WO | 2020-005003 | A1 | 02 January 2020 |
| WO | 2019-191295 | A1 | 03 October 2019 | AR | 115024 | A1 | 18 November 2020 |
| | | | | AU | 2019-244091 | A1 | 08 October 2020 |
| | | | | BR | 112020018709 | A2 | 05 January 2021 |
| | | | | CA | 3094112 | A1 | 03 October 2019 |
| | | | | CL | 2020002477 | A1 | 12 March 2021 |
| | | | | CN | 112154153 | A | 29 December 2020 |
| | | | | CO | 2020013246 | A2 | 10 November 2020 |
| | | | | EA | 202092316 | A1 | 25 May 2021 |
| | | | | EP | 3774861 | A1 | 17 February 2021 |
| | | | | JP | 2021-519094 | A | 10 August 2021 |
| | | | | KR | 10-2020-0136453 | A | 07 December 2020 |
| | | | | PE | 20210313 | A1 | 12 February 2021 |
| | | | | SG | 11202009017 | A | 29 October 2020 |
| | | | | TW | 202003551 | A | 16 January 2020 |
| | | | | US | 10787494 | B2 | 29 September 2020 |
| | | | | US | 2019-0300592 | A1 | 03 October 2019 |
| | | | | US | 2019-0359672 | A1 | 28 November 2019 |
| WO | 2020-113403 | A1 | 11 June 2020 | CN | 112105632 | A | 18 December 2020 |
| | | | | JP | 2021-511348 | A | 06 May 2021 |
| | | | | US | 2021-0052727 | A1 | 25 February 2021 |
| | | | | WO | 2019-144309 | A1 | 01 August 2019 |
| | | | | WO | 2019-147837 | A2 | 01 August 2019 |
| | | | | WO | 2019-147837 | A3 | 31 October 2019 |

Form PCT/ISA/210 (patent family annex) (July 2019)

**REFERENCES CITED IN THE DESCRIPTION**

**Patent documents cited in the description**

- US 5229109 A

- KR 101667096

**Non-patent literature cited in the description**

- **IMAI et al.** *Cancer Sci,* 2007, vol. 98, 416-423
- **ENGELS ; UHLMANN.** *Angew Chem Int Ed Engl.,* 1988, vol. 37, 73-127
- **GILLIES et al.** *J. Immunol. Meth,* 1989, vol. 125, 191-202

- **SAKANO et al.** *Nature,* 1980, vol. 286, 676-683
- **WATSON et al.** *Nucleic Acid Research,* 1984, vol. 12, 5145-5164
- Remington's Pharmaceutical Sciences. 1995